# EUROPEAN PATENT APPLICATION

(11) **EP 4 361 246 A1**
(43) Date of publication of application: **01.05.2024**
(21) Application number: 22828695.1
(22) Date of filing: 20.06.2022
(51) Int. Cl.: C12M 1/34, B01L 7/00, C12M 1/00

(54) **CELL THAWER AND OPERATING METHOD THEREFOR**

(30) Priority: 25.06.2021 KR 20210082930; 27.07.2021 KR 20210098642; 05.11.2021 KR 20210151330
(71) Applicant: Amogreentech Co., Ltd., Gimpo-si, Gyeonggi-do 10014 (KR); Kangstem Biotech Co., Ltd., Seoul 06179 (KR)
(72) Inventor: HAN, Kyoung Ku, Gimpo-si, Gyeonggi-do 10014 (KR); JANG, Seon Ho, Gimpo-si, Gyeonggi-do 10014 (KR); SONG, Jae Kyung, Gimpo-si, Gyeonggi-do 10014 (KR); SEO, In Yong, Gimpo-si, Gyeonggi-do 10014 (KR); KIM, Jae Yun, Gimpo-si, Gyeonggi-do 10014 (KR); KANG, Kyung Sun, Seoul 06338 (KR); LEE, Mi Hye, Seoul 08861 (KR); HEO, Hyun Suk, Daegu 41226 (KR)
(74) Representative: Treeby, Philip David William
(86) International application number: PCT/KR2022/008715
(87) International publication number: WO 2022/270848

(57) **Abstract**

A cell thawer is provided. A cell thawer according to one embodiment of the present invention comprises: a thawing part comprising a heating block, which includes a first heating block and a second heating block that form a heating space for heating a container storing a predetermined amount of a biological material including cells, a heater, which is provided in the heating block so that the heating block can be provided with heat, an insulating block, which is coupled to the heating block so as to encompass the heater, and a support, which is for supporting the lower portion of the container in the heating space; a driving part for providing driving force that linearly moves each of the first heating block and the second heating block in the left and right directions; and a control part for controlling the operations of the thawing part and the driving part.

## Description

### [Technical Field]

The present invention relates to a cell thawer and an operating method therefor.

### [Background Art]

In general, various biological materials are stored at low temperature after being stored in containers such as vials. For example, plasma and tissue cells are stored at a maximum of -100 degrees Celsius, and stem cells are stored at cryogenic temperatures of up to -165 degrees Celsius by using gaseous liquid nitrogen.

These biological materials are tested at a temperature higher than the storage temperature of the aforementioned low-temperature state. Accordingly, a thawing process is required to test the biological material.

To this end, a cell thawer has been proposed for thawing a biological material in a low-temperature state by heating a container such as a vial in which the biological material is stored.

However, conventional cell thawers adopt a method of transferring heat while a heating block and a container are in contact with each other. Accordingly, the conventional cell thawers have a problem in that it is difficult to uniformly heat the container as a whole, and since the high-temperature heating block directly contacts the container, there is a problem in that the container is cracked or deformed by the high temperature.

In addition, the conventional cell thawers have been operated in the manner of simply displaying whether the heating block is operating normally through an indicator such as an LED.

Accordingly, the conventional cell thawers have a problem in that the operating temperature of the heating block cannot be easily measured during qualification evaluation to determine whether the heating block operates correctly.

### [Disclosure]

### [Technical Problem]

The present invention has been devised in view of the above, and an object of the present invention is to provide a cell thawer which is capable of uniformly heating a container including a biological material in the heating process while ensuring stability against heat.

In addition, another object of the present invention is to provide a cell thawer which capable of increasing the efficiency of energy usage.

Moreover, still another object of the present invention is to provide a cell thawer which is capable of conveniently measuring the operating temperature of a heating block during qualification evaluation.

### [Technical Solution]

In order to solve the above-described problems, the present invention provides a cell thawer, including a thawing part including a heating block which includes a first heating block and a second heating block that form a heating space for heating a container storing a predetermined amount of a biological material including cells, a heater which is installed in the heating block to provide heat to the heating block, a heat insulating block which is coupled to the heating block so as to encompass the heater, and a pedestal which is for supporting a lower portion of the container in the heating space; a driving part for providing a driving force that linearly moves each of the first heating block and the second heating block in the left and right directions; and a control part for controlling the operations of the thawing part and the driving part.

In addition, the present invention provides a method for operating a cell thawer including a first heating block and a second heating block that form a heating space for accommodating a container storing a predetermined amount of a biological material including cells through linear movement by a driving force of a motor, and heating the container accommodated in the heating space through heat provided from a heater, the method including a first step of preheating the first heating block and the second heating block to a predetermined temperature by driving the heater while one surface of the first heating block and one surface of the second heating block facing each other are in contact with each other; a second step of heating the container which is placed in the heating space so as to thaw the biological material while the first heating block and the second heating block are changed to a heating position forming the heating space; and a third step of changing the first heating block and the second heating block to a standby position by moving the same away from the heating position such that the container placed in the heating space can be removed, wherein in the second step, the container placed in the heating space is heated by heat transferred from the first heating block and the second heating block in a non-contact manner while not contacting the first heating block and the second heating block.

### [Advantageous Effects]

According to the present invention, the container can be uniformly heated by heating the container in a non-contact heating manner by using radiant heat, and it is possible to prevent cracks or thermal deformation of the container that may occur when the container is heated by conductive heat.

In addition, according to the present invention, since the temperature of the heating block can be maintained to be constant, a plurality of containers can be sequentially thawed. Through this, the present invention can improve energy consumption efficiency because it is possible to thaw a plurality of containers while reducing the reuse waiting time for thawing the containers.

Furthermore, according to the present invention, when the upper cover is separated, the sensor insertion hole for qualification evaluation is exposed to the outside such that qualification evaluation can be easily performed.

### [Description of Drawings]

FIG. 1 is a view showing a cell thawer according to one embodiment of the present invention;
FIG. 2 is a view of FIG. 1 viewed from another direction;
FIG.3 is a view in which the housing is separated from FIG. 1;
FIG.4 is a view showing the cell thawer according to the first embodiment of the present invention, showing a state in which the housing and circuit board are removed from FIG.3;
FIG. 5 is a view of FIG. 4 viewed from another direction;
FIG. 6 is a view in which the thawing part is separated from FIG. 4;
FIG. 7 is a view in which the thawing part is extracted and separated from FIG. 6;
FIG.8 is a view taken from the driving part in FIG.6;
FIG. 9 is an enlarged view of a part of FIG. 5, in which portions of the first heating block and the second heating block are cut in a standby position;
FIG. 10 is a view showing a portion of the cell thawer according to the first embodiment of the present invention in a state where the heating block is disposed at a heating position;
FIG. 11 is a view showing the arrangement relationship of sensors and heating blocks corresponding to the state of FIG. 10, showing a state in which the first heating block, the first heat insulating block and the first guide block are removed;
FIG. 12 is an operating state diagram of the cell thawer according to the first embodiment of the present invention, and is a partial cross-sectional view of the standby position when initially driven, viewed from the A-A direction in FIG. 10;
FIG. 13 is an operating state diagram showing a state in which the heating block is preheated in the cell thawer according to the first embodiment of the present invention, and is a partial cross-sectional view as viewed from the A-A direction of FIG. 10;
Figure 14 is an operating state diagram showing the process of loading a container into the cell thawer according to the first embodiment of the present invention, and is a partial cross-sectional view viewed from the direction A-A of FIG. 10;
FIG. 15 is an operating state diagram of the cell thawer according to the first embodiment of the present invention, and is a partial cross-sectional view of a state in which the container is heated in a heating position, as viewed from the A-A direction in FIG. 10;
FIG. 16 is an operating state diagram of the cell thawer according to the first embodiment of the present invention, and is a partial cross-sectional view of the process of removing the container from the standby position, as viewed from the A-A direction of FIG. 10;
FIG. 17 is a view showing a cell thawer according to the second embodiment of the present invention, showing a state in which the top cover is separated from the state in which the cover is removed in FIG. 1;
FIG. 18 is a cross-sectional view taken in the B-B direction of FIG. 17, extracting the thawing part;
FIG. 19 is a view showing the cell thawer according to the second embodiment of the present invention, showing a state in which the housing and circuit board are removed from FIG. 3;
FIG. 20 is a view of FIG. 19 viewed from another direction;
FIG. 21 is a view in which the thawing part is separated from FIG. 19;
FIG. 22 is a view in which the thawing part is extracted and separated in FIG. 21;
FIG. 23 is a view in which the driving part is extracted in FIG. 21;
FIG. 24 is an enlarged view of a portion of FIG. 20, in which portions of the first heating block and the second heating block are cut in a standby position;
FIG. 25 is a view showing a portion of the cell thawer according to the second embodiment of the present invention in a state where the heating block is disposed at the heating position;
FIG. 26 is a view showing the arrangement relationship of sensors and heating blocks corresponding to the state of FIG. 25, showing a state in which the first heating block, the first heat insulating block and the first guide block are removed;
FIG. 27 is an operating state diagram of the cell thawer according to the second embodiment of the present invention, and is a partial cross-sectional view of the standby position when initially driven, viewed from the C-C direction in FIG. 25;
FIG. 28 is an operating state diagram showing a state in which the heating block is preheated in the cell thawer according to the second embodiment of the present invention, and is a partial cross-sectional view viewed from the C-C direction of FIG. 25;
FIG. 29 is an operating state diagram showing the process of loading a container into the cell thawer according to the second embodiment of the present invention, and is a partial cross-sectional view viewed from the C-C direction of FIG. 25;
FIG. 30 is an operating state diagram of the cell thawer according to the second embodiment of the present invention, and is a partial cross-sectional view of the state in which the container is heated in the heating position, as viewed from the C-C direction in FIG. 25;
FIG. 31 is an operating state diagram of the cell thawer according to the second embodiment of the present invention, and is a partial cross-sectional view of the process of taking out the container from a standby position, as viewed from the C-C direction in FIG. 25; and
FIG. 32 is a block diagram showing the method for operating a cell thawer according to one embodiment of the present invention.

### [Modes of the Invention]

Hereinafter, with reference to the accompanying drawings, the exemplary embodiments of the present invention will be described in detail so that those skilled in the art can easily practice the present invention. The present invention may be embodied in many different forms and is not limited to the exemplary embodiments set forth herein. In order to clearly describe the present invention in the drawings, parts that are irrelevant to the description are omitted, and the same reference numerals are assigned to the same or similar components throughout the specification.

The cell thawers 100, 200 according to one embodiment of the present invention may heat and thaw a biological material in a low-temperature state (e.g., frozen state) contained in the container 10.

Herein, the biological material may include cells, which are the structural basic unit of an organism, and the container 10 may be a known laboratory container or medical container such as a vial, a beaker or a test tube.

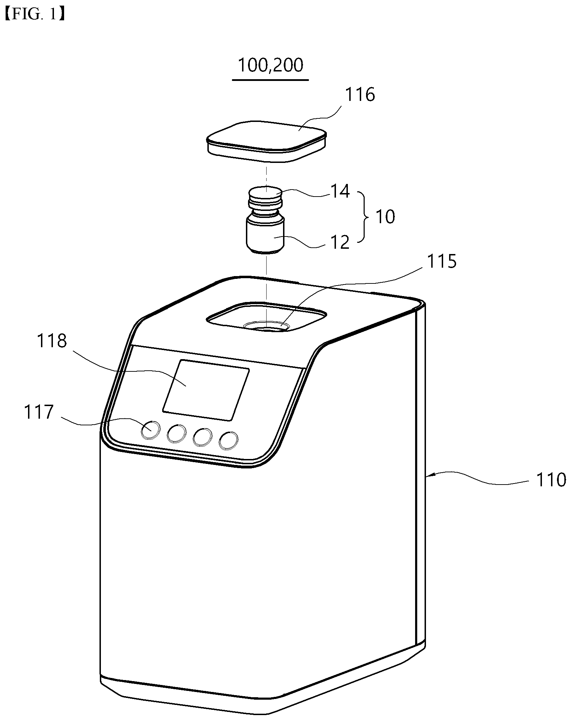

In addition, as illustrated in FIG. 1, the container 10 may include a container body 12 in which the biological material is accommodated and a cap part 14 which covers an open top portion of the container body 12, and approximately 6 mL or 10 mL of a biological material may be contained in the container body 12. However, the shape of the container 10 and the capacity of the biological material contained in the container body 12 are not limited thereto, and may be changed into various shapes depending on design conditions.

In this case, the cell thawers 100, 200 according to one embodiment of the present invention heats the container 10 uniformly as a whole by heating the container 10 in a non-contact heating manner through radiant heat and/or convection heat, and it is possible to prevent cracks or thermal deformation of the container 10 that may occur during contact heating by conductive heat in advance.

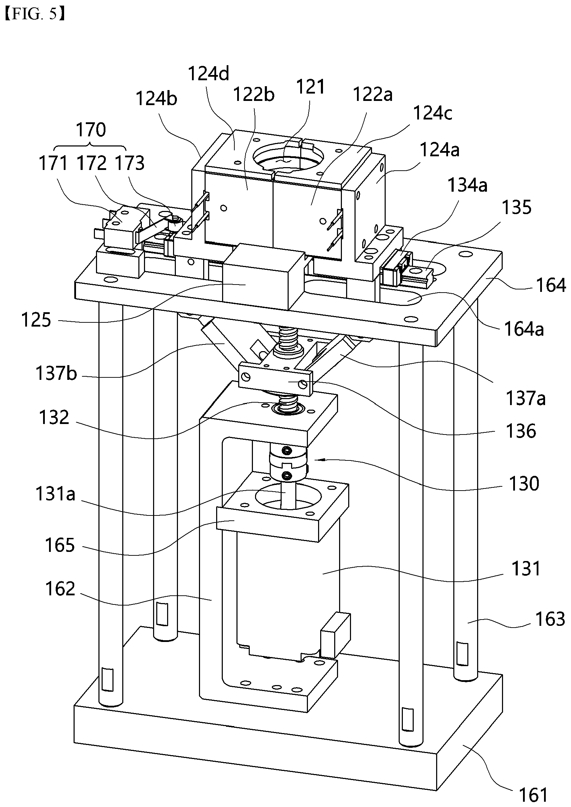

To this end, as illustrated in FIGS. 1 to 6 and 17 to 21, the cell thawers 100, 200 according to one embodiment of the present invention includes a housing 110, a thawing part 120, a driving part 130 and a control part 150.

As illustrated in FIGS. 1 to 3, the housing 110 may form an outer surface of the cell thawers 100, 200, and it may be formed in a box shape such that the thawing part 120, the driving part 130 and the control part 150 may be placed therein.

The housing 110 may be composed of one member or may be composed of a plurality of members, and the plurality of members may be detachably coupled.

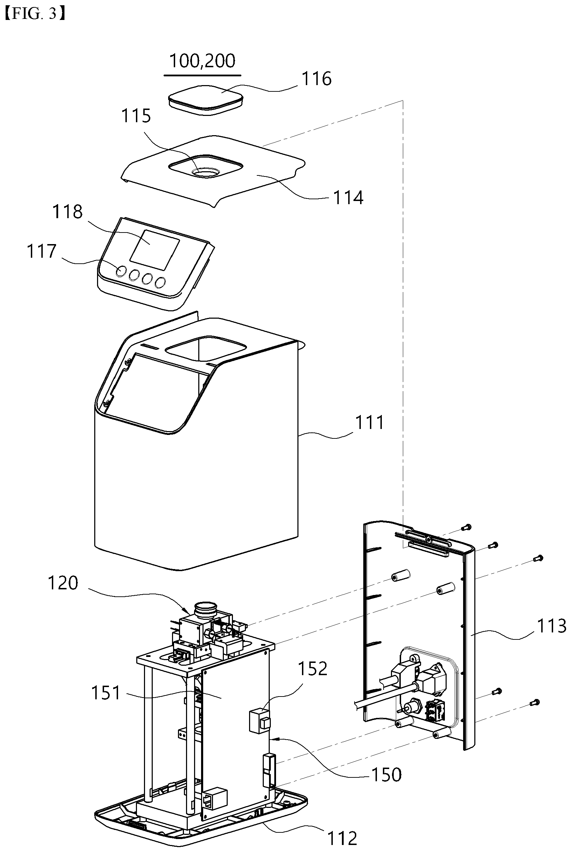

For example, as illustrated in FIG. 3, the housing 110 may include a box-shaped main body 111, a lower cover 112 for covering an open lower portion of the main body 111, a rear cover 113 for covering an open rear surface of the main body 111, and an upper cover 114 for covering the top of the main body 111.

In this case, the thawing part 120, the driving part 130 and the control part 150 may be disposed inside the main body 111.

In this case, the housing 110 may include an inlet 115 which is formed to penetrate a predetermined area such that the container 10 can be inserted into the body 111, and the inlet 115 may be covered through a cover 116.

Herein, the inlet 115 may be formed to penetrate the housing 110 so as to be located at a position corresponding to a heating space 121 to be described below.

For example, the inlet 115 may be formed to penetrate the upper cover 114 so as to communicate with a heating space 121 formed in the thawing part 120 at a heating position to be described below. Through this, when the container 10 is inserted into the inlet 115, the cap part 14 of the container 10 may be exposed to the outside through the inlet 115, and the remaining part except for the cap part 14 may be inserted into the side of the heating space 121.

In addition, as illustrated in FIG. 1, a manipulation part 117 for manipulating the operation of the cell thawer 100, 200 according to one embodiment of the present invention, and a display 118 for displaying the operating state of the cell thawers 100, 200 may be provided on one side of the housing 110.

Such a manipulation part 117 may be an interface for transmitting an input signal to the control part 150 through user manipulation.

Herein, the manipulation part 117 may be configured with a known press-type physical button or capacitive touch button.

In addition, the manipulation part 117 may be provided separately from the display 118, or may be provided in an integrated form with the display 118.

Moreover, the display 118 may output information on the overall operating state of the cell thawers 100, 200 operated through the control part 150 (e.g., the temperature of the heating blocks 122a, 122b, and the surface temperature of the container 10, the operation status such as thawing and completion of thawing, and thawing progress time), date and time at the time of thawing, an error message such as sensor or heater failure, which will be described below, and the like.

Through this, the user may transmit input signals, such as the operation and stop of the cell thawers 100, 200, thawing temperature adjustment, thawing time adjustment and the like, to the control part 150 through the manipulation of the manipulation part 117, and the display 118 may output information on the overall state of the cell thawers 100, 200 through the control part 150.

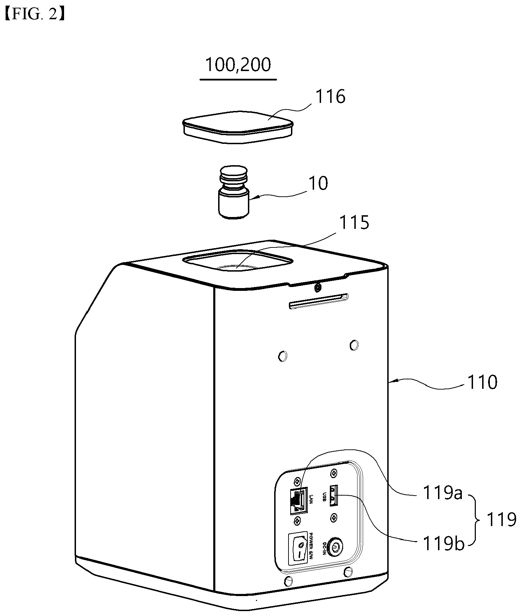

Moreover, as illustrated in FIG. 2, the cell thawers 100, 200 may include at least one communication port 119 provided to be exposed to the outside from one side of the housing 110, and the communication port 119 may be electrically connected to the control part 150.

For example, the communication port 119 may include a LAN port 119a for communication with communication device such as an external network device or a PC, and a USB port 119b for inputting or outputting data, and the communication port 119 may be mounted on the rear cover 113 so as to be electrically connected to the control part 150.

In this case, when the cell thawers 100, 200 according to one embodiment of the present invention is connected to the external network device or communication device such as a PC through the communication port 119, the control part 150 may be time-synchronized with communication device such as the external network device or PC.

For example, when the cell thawers 100, 200 are connected to a PC such as a laptop computer, the cell thawers 100, 200 may be synchronized with the date and time of the PC by using a synchronization program installed in the PC.

Through this, the cell thawers 100, 200 according to one embodiment of the present invention may be synchronized with the local time of a region where thawing is performed when in use, and the local time of the region may be output through the display 118.

As a result, the cell thawers 100, 200 according to one embodiment of the present invention may store history information about the exact time of thawing of the biological material contained in the container 10, and the user may confirm the exact thawing time of the biological material contained in the container 10 though the history information.

Moreover, information related to thawing may be stored in the cell thawer 100, 200 itself or may be stored in a synchronization program of a communication device that is connected through the communication port 119.

In this case, although the information related to thawing stored in the cell thawers 100, 200 or the synchronization program may be stored by date, a thawing history may be stored for each thawing case, and the thawing history may include the local time of a region where the thawing is performed.

The thawing part 120 may heat the container 10 in a state where the container 10 introduced into the housing 110 through the inlet 115 is accommodated in the heating space 121.

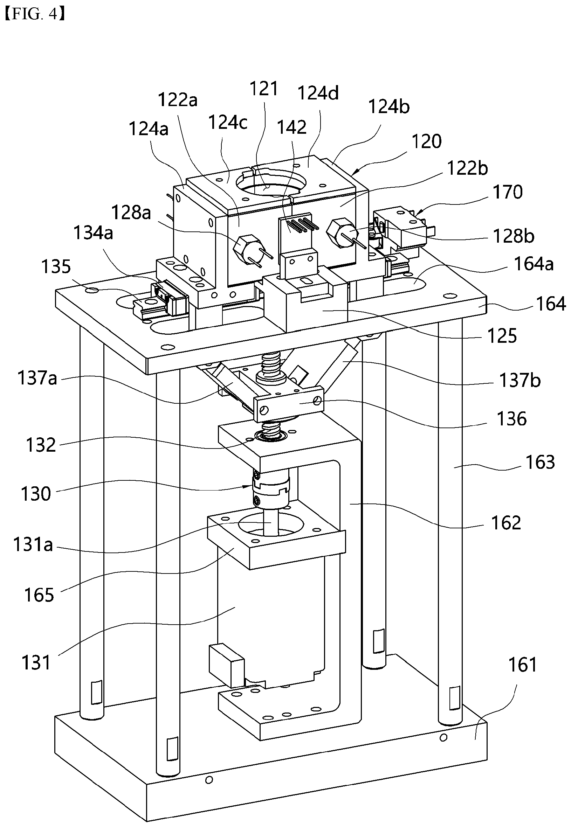

To this end, as illustrated in FIGS. 4 to 7 and 19 to 22, the thawing part 120 may include heating blocks 122a, 122b for forming a heating space 121 for accommodating and heating the container 10, heaters 123a, 123b which are installed in the heating blocks 122a, 122b to provide heat to the heating blocks 122a, 122b, heat insulating blocks 124a, 124b which are coupled to the heating blocks 122a, 122b so as to surround the heaters 123a, 123b, and a pedestal 125 for supporting a lower portion of the container 10 in the heating space 121.

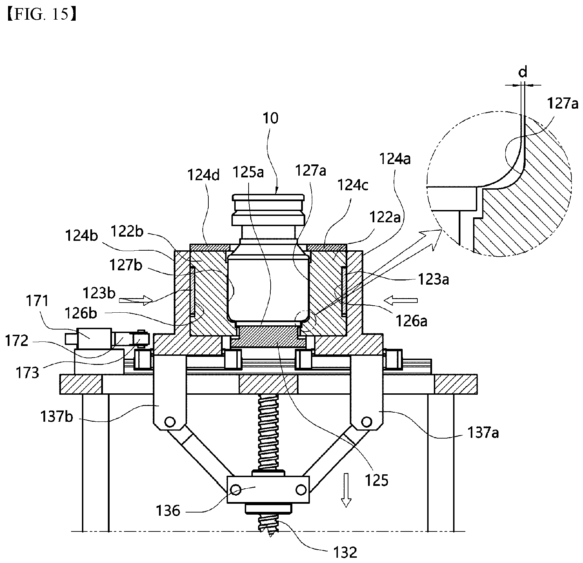

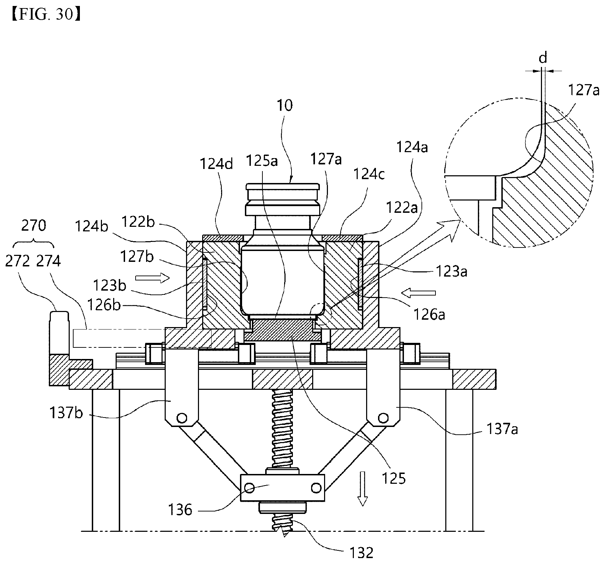

Accordingly, when the container 10 is introduced into the housing 110 through the inlet 115, the lower portion of the container 10 may be supported through the pedestal 125, and the container 10 may be surrounded by the heating blocks 122a, 122b while being accommodated in the heating space 121 (refer to FIGS. 15 and 30).

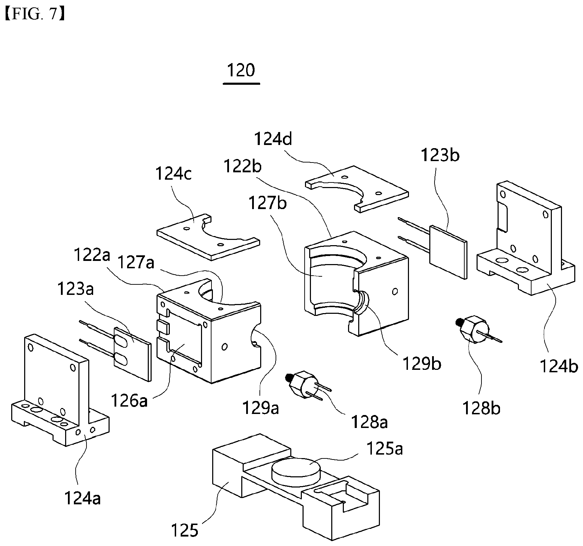

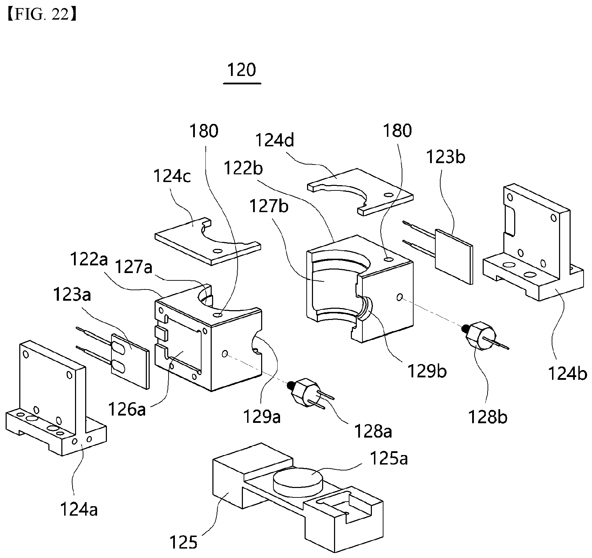

In this case, as illustrated in FIGS. 7 and 22, the heating blocks 122a, 122b may include arrangement grooves 126a, 126b that are formed to be drawn inwardly on one surface, and the heaters 123a, 123b may be inserted into the arrangement grooves 126a, 126b, and the heat insulating blocks 124a, 124b may be coupled to the heating blocks 122a, 122b so as to completely cover the arrangement grooves 126a, 126b while the heaters 123a, 123b are inserted into the placement groove 126a, 126b.

In addition, the cell thawers 100, 200 according to one embodiment of the present invention may include auxiliary heat insulating blocks 124c, 124d to block heat transferred from the heaters 123a, 124b from being discharged to the outside, and the auxiliary heat insulating blocks 124c, 124d may be coupled to the heating blocks 122a, 122b to cover the upper surfaces of the heating blocks 122a, 122b.

Herein, the heating blocks 122a, 122b may be made of a material having thermal conductivity such as metal, and the heat insulating blocks 124a, 124b and the auxiliary heat insulating blocks 124c, 124d may be made of a material having heat insulating properties.

Accordingly, when the heaters 123a, 123b generate heat, the heating blocks 122a, 122b may be heated by the heat generated from the heaters 123a, 123b, and the heat generated from the heaters 123a, 123b may be concentrated toward the heating blocks 122a, 122b by limiting the direction of heat movement through the heat insulating blocks 124a, 124b and the auxiliary heat insulating blocks 124c, 124d, and the heat transferred from the heaters 123a, 123b to the heating blocks 122a, 122b may be transferred toward the heating space 121.

As a result, the container 10 may be heated by the heat provided from the heating blocks 122a, 122b, and the biological material stored in the container 10 may be thawed by the heat.

Moreover, since the heat generated from the heaters 123a, 123b may be concentrated toward the heating blocks 122a, 122b by limiting the direction of heat movement through the heat insulating blocks 124a, 124b and the auxiliary heat insulating blocks 124c, 124d, the energy consumption efficiency of the cell thawers 100, 200 according to one embodiment of the present invention may be improved.

In addition, since the container 10 accommodated in the heating space 121 may be heated by using the heat of the heating blocks 122a, 122b heated to a constant temperature by the heat transferred from the heaters 123a, 123b, the circumferential surface of the container 10 may be heated to a uniform temperature. Herein, the circumferential surface of the container 10 may be the circumferential surface of the container body 12 excluding the cap part 14 and the lower surface.

In this case, as illustrated in FIGS. 7 and 22, the thawing part 120 may include temperature sensors 128a, 128b which are installed on the heating blocks 122a, 122b to measure the temperature of the heating blocks 122a, 122b. Herein, the temperature sensors 128a, 128b may be known contact temperature sensors such as thermocouples, resistance thermometers (RTDs) and thermistors, and the temperatures of the heating blocks 122a, 122b measured through the temperature sensors 128a, 128b may be transmitted to the control part 150.

Accordingly, as the control part 150 controls the driving of the heaters 123a, 123b based on the temperature information measured by the temperature sensors 128a, 128b, it is possible to maintain the temperatures of the heating blocks 122a, 122b to constant temperatures. For example, the control part 150 may maintain the temperature of the heating blocks 122a, 122b to be constant by controlling the driving of the heaters 123a, 123b through PID control.

Moreover, the control part 150 may prevent overheating of the heating blocks 122a, 122b by controlling the driving of the heaters 123a, 123b based on the temperature information measured by the temperature sensors 128a, 128b.

In the present invention, the heaters 123a, 123b may be provided as ceramic heaters to secure reliability and improve the lifespan cycle of products even under operating conditions in which heating and cooling are repeatedly performed, but the present invention is not limited thereto, and a variety of heaters may all be applied.

Meanwhile, the heating space 121 may be provided in a shape corresponding to the shape of the container 10, and the heating space 121 may be formed through at least two heating blocks 122a, 122b.

As a specific example, as illustrated in FIGS. 7 and 22, the heating blocks 122a, 122b may include a first heating block 122a and a second heating block 122b that are disposed such that surfaces thereof face each other, and the heating space 121 may be formed through a pair of opposing surfaces 127a, 127b that are formed by being drawn in on surfaces thereof facing each other in the first heating block 122a and the second heating block 122b.

Herein, the pair of opposing surfaces 127a, 127b may be drawn inward to have a shape corresponding to the circumferential surface of the container 10.

For example, when the container 10 is formed in the shape of a cylinder, the pair of opposing surfaces 127a, 127b may be arc-shaped curved surfaces, and when the container 10 is formed in the shape of a square column, the pair of opposing surfaces 127a, 127b may be formed as bent surfaces having a substantially 'c' cross-sectional shape.

Accordingly, when the container 10 is introduced into the heating space 121 through the inlet 115, the container 10 may be completely surrounded by a pair of opposing surfaces 127a, 127b whose circumferential surface defines the heating space 121.

As a result, the circumferential surface of the container 10 accommodated in the heating space 121 may be uniformly heated by the heat provided from the first heating block 122a and the second heating block 122b.

In this case, each of the first heating block 122a and the second heating block 122b may include arrangement grooves 126a, 126b that are formed to be drawn inward on one surface, and the heaters 123a, 123b may include a first heater 123a and a second heater 123b that are inserted into the arrangement groove 126a formed in the first heating block 122a and the arrangement groove 126b formed in the second heating block 122b, respectively.

In addition, the heat insulating blocks 124a, 124b may include a first heat insulating block 124a and a second heat insulating block 124b, and the first heat insulating block 124a may be coupled to the first heating block 122a so as to completely cover the arrangement groove 126a while the first heater 123a is inserted into the arrangement groove 126a of the first heating block 122a, and the second heat insulating block 125a may be coupled to the second heating block 122b so as to completely cover the arrangement groove 126b while the second heater 123b is inserted into the arrangement groove 126b of the second heating block 122b.

Moreover, the auxiliary heat insulating blocks 124c, 124d may include a first auxiliary heat insulating block 124c which is fastened to an upper surface of the first heating block 122a and a second auxiliary heat insulating block 124d which is fastened to an upper surface of the second heating block 122b.

In addition, the temperature sensors 128a, 128b may include a first temperature sensor 128a which is installed on the first heating block 122a to measure the temperature of the first heating block 122a and a second temperature sensor 128b which is installed on the second heating block 122b to measure the temperature of the second heating block 122b.

Accordingly, the heat generated from the first heater 123a may heat the first heating block 122a, and the heat generated from the second heater 123b may heat the second heating block 122b. In addition, the control part 150 may control the driving of the first heater 123a and the second heater 123b based on the temperature information measured by the first temperature sensor 128a and the second temperature sensor 128b.

Through this, the control part 150 may maintain the temperature of the first heating block 122a and the second heating block 122b to be constant.

In this case, the control part 150 may control the driving of the first heater 123a and the second heater 123b together based on the temperature information measured through the first temperature sensor 128a and the second temperature sensor 128b, or the control part 150 may individually control the driving of the first heater 123a and the second heater 123b based on temperature information measured through the first temperature sensor 128a and the second temperature sensor 128b.

Through this, even if the heating blocks 122a, 122b include the first heating block 122a and the second heating block 122b in the cell thawers 100, 200 according to one embodiment of the present invention, the temperatures of the first heating block 122a and the second heating block 122b may be preciously controlled through the control of the control part 150.

Accordingly, since the first heating block 122a and the second heating block 122b may be maintained at the same temperature without temperature deviation through the control part 150, the circumferential surface of the container 10 may be uniformly heated in the heating space 121.

Moreover, when the control part 150 individually controls the driving of the first heater 123a and the second heater 123b, the user may immediately check an abnormality of the corresponding part based on the information output through the display 118, and take appropriate action on the corresponding part in which the abnormality has occurred.

For example, if any one of the heaters 123a, 123b respectively installed in the first heating block 122a and the second heating block 122b, or any one of the temperature sensors 128a, 128b respectively installed in the first heating block 122a and the second heating block 122b does not operate normally, the user may immediately check an abnormality of the corresponding part based on the information output through the display 118, and take appropriate action on the corresponding part in which the abnormality has occurred.

Meanwhile, in the cell thawers 100, 200 according to one embodiment of the present invention, the container 10 accommodated in the heating space 121 may be heated by heat transferred in a non-contact manner from the heating blocks 122a, 122b while not in contact with the heating block 122a, 122b.

For example, while the container 10 is inserted into the heating space 121, the circumferential surface of the container 10 may be spaced apart at a predetermined interval with the opposing surface 127a of the first heating block 122a and the opposing surface 127b of the second heating block 122b forming the heating space 121.

In this case, the lower surface of the container 10 may be supported through a protruding member 125a which is formed to protrude from one surface of the pedestal 125 at a predetermined height.

That is, as illustrated in FIGS. 15 and 30, while the container 10 is inserted into the heating space 121 and the lower portion of the container 10 is supported through the protruding member 125a, a gap (d) may be formed between the pair of opposing surfaces 127a, 127b and the outer surface of the container 10.

Accordingly, the heat stored in the first heating block 122a and the second heating block 122b may be transferred to the relatively low-temperature container 10 through convection and/or radiation instead of conduction.

Therefore, since the cell thawers 100, 200 according to one embodiment of the present invention may heat the circumferential surface of the container 10 through heat transfer using convection and/or radiation, the circumferential surface of the container 10 may be uniformly heated through convective heat and/or radiant heat transferred from the heating blocks 122a, 122b.

That is, compared to the case where the container 10 and the heating blocks 122a, 122b are in direct contact and the container 10 is heated through conduction heat, the cell thawers 100, 200 according to one embodiment of the present invention may heat the circumferential surface of the container 10 through convective heat and or radiant heat, thereby preventing the concentration of heat in a local location of the container 10.

Moreover, in the cell thawers 100, 200 according to one embodiment of the present invention, even if the container 10 is made of a glass or plastic material that is vulnerable to high temperature heat, the first heating block 122a and the second heating block 122b are not in direct contact with the container 10, and thus, it is possible to prevent cracks or thermal deformation of the container that may occur due to heat when in direct contact with a high-temperature object.

In the present invention, the gap (d) between the pair of opposing surfaces 127a, 127b forming the heating space 121 and the outer surface of the container 10 may be 0.2 mm to 0.3 mm, but the present invention is limited thereto, and the size of the gap (d) may be appropriately changed according to the overall size of the container 10.

In this case, at least any one of the first heating block 122a and the second heating block 122b may be reciprocally moved in a straight line, and the heating space 121 may be formed when the first heating block 122a and the second heating block 122b are arranged to be located close to each other through a straight-line movement of at least any one of the first heating block 122a and the second heating block 122b. The detailed description thereof will be described below.

Meanwhile, as illustrated in FIGS. 7, 9, 22 and 24, the pedestal 125 for supporting a lower portion of the container 10 may include a protruding members 125a which is formed to protrude at a predetermined height so that the lower portion of the container 10 is spaced apart from one surface of the pedestal 125 by a predetermined height.

When the container 10 is inserted into the inlet 115, the protruding member 125a may support the lower surface of the container 10 such that the lower surface of the container 10 is spaced apart from one surface of the pedestal 125 by a predetermined height. Through this, the circumferential surface of the container 10 may be positioned in the heating space 121 at a position facing a pair of opposing surfaces 127a, 127b forming the heating space 121.

Accordingly, the container 10 may be smoothly heated by the heat transferred through the pair of opposing surfaces 127a, 127b while being disposed in the heating space 121, and the circumferential surface of the container 10 disposed in the heating space 121 may maintain a uniform gap with the pair of opposing surfaces 127a, 127b.

The driving part 130 may linearly move the heating blocks 122a, 122b in left and right directions. For example, as described above, when the heating blocks 122a, 122b include the first heating block 122a and the second heating block 122b, the driving part 130 may move at least any one of the first heating block 122a and the second heating block 122b in a straight line in the left and right directions.

As a non-limiting example, when the heating blocks 122a, 122b include the first heating block 122a and the second heating block 122b, the driving part 130 may move the first heating block 122a and the second heating block 122b in a straight line in the left and right directions, respectively.

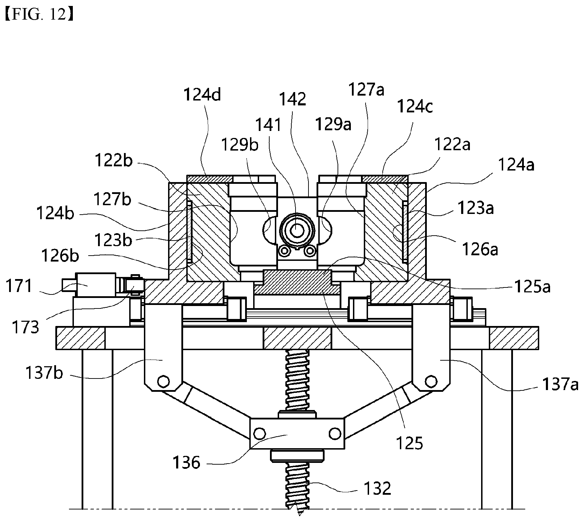

Through this, in the cell thawers 100, 200 according to one embodiment of the present invention, the first heating block 122a and the second heating block 122b may be changed to a heating position in which they are disposed to be close to each other through the driving of the driving part 130 (refer to FIGS. 15 and 30), and a standby position in which they are disposed to be are spaced apart from each other at intervals (refer to FIGS. 12, 14, 16, 27, 29 and 31).

Accordingly, as illustrated in FIGS. 15 and 30, when the first heating block 122a and the second heating block 122b are located in the heating position, a heating space 121 for accommodating and heating the container 10 may be formed between the opposing surface 127a of the first heating block 122a and the opposing surface 127b of the second heating block 122b.

In addition, as illustrated in FIGS. 12, 14, 16, 27, 29 and 31, when the first heating block 122a and the second heating block 122b are located in the standby position, a gap between the opposing surface 127a of the first heating block 122a and the opposing surface 127b of the second heating block 122b may be widened. Through this, the user may insert the container 10 to be disposed between the first heating block 122a and the second heating block 122b or remove the container 10 that is disposed between the first heating block 122a and the second heating block 122b.

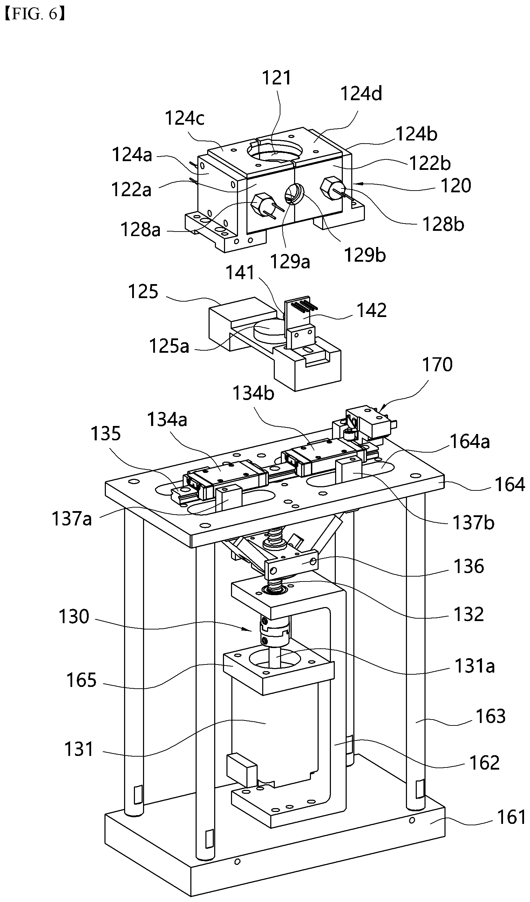

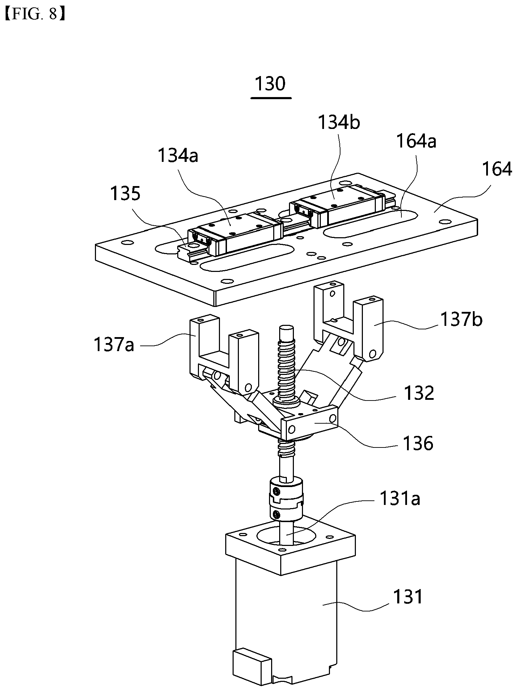

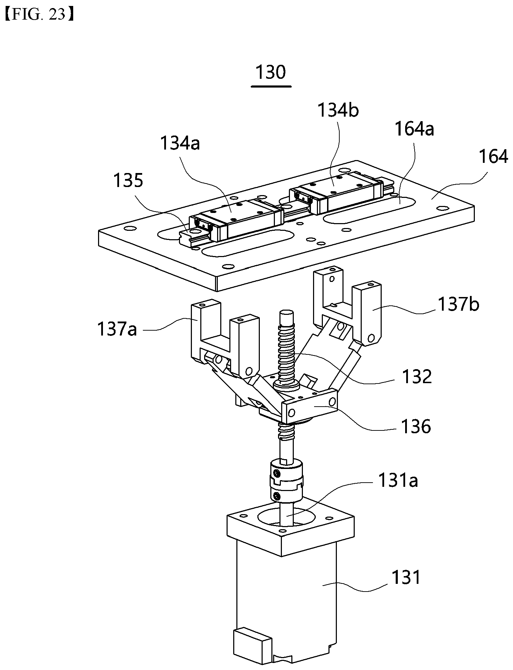

To this end, as illustrated in FIGS. 6, 8, 21 and 23, the driving part 130 may include a motor 131 for providing a driving force, a rotating member 132 which is axially coupled to a rotating shaft 131a of the motor 131, a moving member 136 which moves up and down along the rotating member 132 when the rotating member 132 rotates, and a power transmission member which interconnects the rotating member 132 and the thawing part 120 such that the first heating block 122a and the second heating block 122b can be moved linearly in the left and right directions, respectively, through the driving force of the motor 131.

In addition, the power transmission member may include guide blocks 134a, 134b which are fixedly coupled to the heat insulating blocks 124a, 124b, a guide rail 135 for guiding linear movement of the guide blocks 134a, 134b, and link members 137a, 137b for connecting the guide blocks 134a, 134b and the moving member 136 to each other.

As a specific example, the guide blocks 134a, 134b may include a first guide block 134a which is fixedly coupled to the first heat insulating block 124a and a second guide block 134b which is fixedly coupled to the second heat insulating block 124b, and the first guide block 134a and the second guide block 134b may be slidably coupled to the guide rail 135.

In addition, the link members 137a, 137b may include a first link member 137a for linking the moving member 136 and the first guide block 134a to each other, and a second link member 137b for linking the moving member 136 and the second guide block 134b to each other.

In this case, the cell thawers 100, 200 according to one embodiment of the present invention may further include a base 161, a mounting table 162, a support bar 163 and a support 164 that are disposed inside the housing 110.

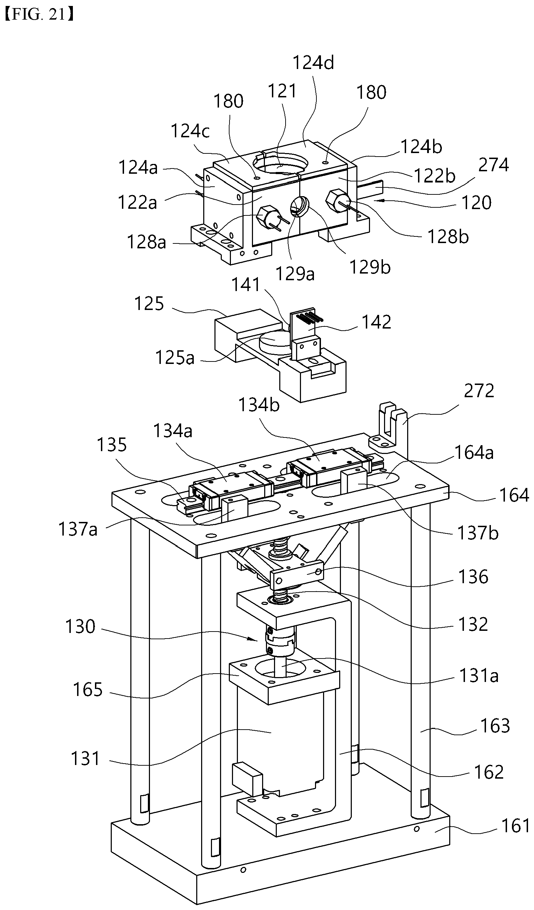

That is, as illustrated in FIGS. 6 and 21, the mounting table 162 may be fixedly coupled to the upper surface of the base 161, and the motor 131 may be fixedly coupled to one side of the mounting table 162 via a coupling base 165, and the rotating member 132 which is axially coupled to a rotating shaft 131a of the motor 131 may be rotatably coupled to the mounting table 162.

In addition, the plate-shaped support 164 having a predetermined area may be disposed in a state of being spaced apart from the base 161 by a predetermined interval upward through at least one support bar 163 having a predetermined length and having one end fixedly coupled to the base 161, and the guide rail 135 may be fixedly coupled to one surface of the support 164.

Moreover, the first guide block 134a and the second guide block 134b may be slidably coupled to the guide rail 135, and the first heat insulating block 124a may be fixedly coupled to the first guide block 134a, and the second heat insulating block 124b may be fixedly coupled to the second guide block 134b.

Herein, the rotating member 132 may be a screw bar having a predetermined length and having one end axially coupled to a rotating shaft 131a of the motor 131, and the moving member 136 may be movably screw-coupled to the rotating member 132.

In addition, the support 164 may include a through-hole 164a having a long hole through which the first link member 137a and the second link member 137b may respectively pass.

In addition, both ends of the first link member 137a may be linked to the rotation member 132 and the first guide block 134a, respectively, and both ends of the second link member 137b may be linked to the rotation member 132 and the second guide block 134b, respectively.

Accordingly, when the rotating member 132 which is axially coupled to the rotating shaft 131a rotates when the motor 131 is driven, as illustrated in FIGS. 12 to 16 and 27 to 31, the moving member 136 may be ascended and descended by screw movement along the rotating member 132, and the first guide block 134a and the second guide block 134b which are each linked to the moving member 136 may be linearly moved in the left and right directions along the guide rail 136 through the ascending and descending of the moving member 136.

As a result, the first heat insulating block 124a and the second heat insulating block 124b which are fixedly coupled to the first guide block 134a and the second guide block 134b, respectively, may be linearly moved in the left and right directions in the same manner as the first guide block 134a and the second guide block 134b, and the first heating block 122a and the second heating block 122b which are fixedly coupled to the first heat insulating block 124a and the second heat insulating block 124b, respectively, may also be linearly moved in the left and right directions.

Through this, in the cell thawers 100, 200 according to one embodiment of the present invention, the first heating block 122a and the second heating block 122b may be changed to heating position in which the first heating block 122a and the second heating block 122b are disposed to be close to each other through the driving of the driving part 130, and a standby position in which the first heating block 122a and the second heating block 122b are disposed to be spaced apart from each other.

Moreover, when the cell thawers 100, 200 according to one embodiment of the present invention are changed from a standby position to a heating position through the control of the control part 140, the lower portion of the container 10 which is accommodated in the heating space 121 may be located at a relatively higher position than the lower surfaces of the first heating block 122a and the second heating block 122b through the protruding member 125a.

Accordingly, the container 10 which is disposed in the heating space 121 may be disposed in a state where the circumferential surface thereof faces the opposing surfaces forming the heating space 121.

The drawings and description illustrate and describe that the rotational movement of the motor 131 is changed into the linear movement of the first guide block 134a and the second guide block 134b through mutual coupling of the rotating member 132, the moving member 136 and the link members 137a, 137b, but the present invention is not limited thereto, and various known structures such as a ball screw structure may all be applied as long as the rotational movement can be converted into linear movement.

In this case, the cell thawers 100, 200 according to one embodiment of the present invention may further include a position detecting means for detecting the position of the heating blocks 122a, 122b.

For example, in the cell thawer 100 according to one embodiment of the present invention, the position detecting means may be a limit switch 170 for detecting the position of the heating blocks 122a, 122b through contact with the heat insulating blocks 124a, 124b.

As illustrated in FIG. 6, such a limit switch 170 may be installed on one surface of the support 164, and may detect the position of the second heating block 122b through contact with the second heat insulating block 124b, and the information detected through the limit switch 170 may be provided toward the control part 150.

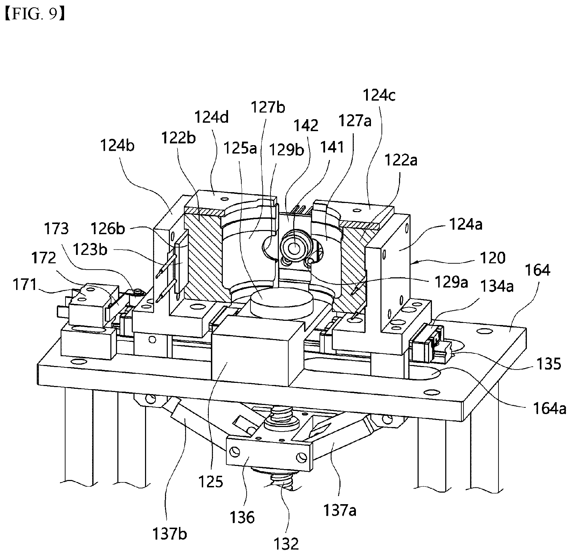

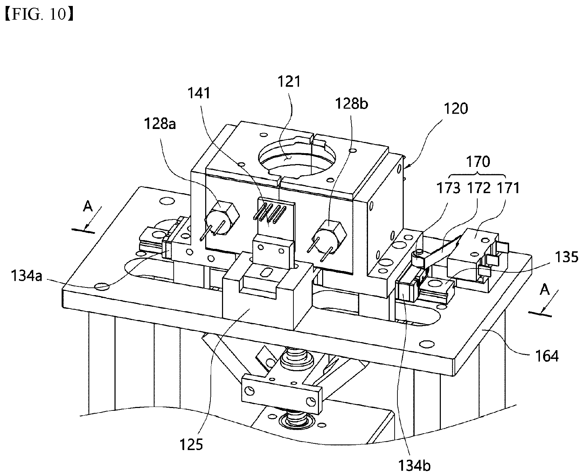

As a specific example, as illustrated in FIGS. 9 and 10, the limit switch 170 may include a switch box 171 which is fixedly installed on one surface of the support 164, an operating lever 172 which is operated by a hinge on one side of the switch box 171, and a roller 173 which is rotatably coupled to one end of the operating lever 172.

Accordingly, in the limit switch 170, when the roller 173 contacts the second heat insulating block 124b and the operation lever 172 operates, the switch box 171 may be operated, and information on whether the switch box 171 is operated may be transmitted toward the control part 150.

Specifically, as described above, when the first heating block 122a and the second heating block 122b are changed from the heating position to the standby position through the driving of the driving part 130, the second heat insulating block 124b may move together with the second heating block 122b.

Through this, the second heat insulating block 124b may be in contact with the roller 173, and as illustrated in FIG. 9, when the second heating block 122b is completely moved to the standby position, the operating lever 172 is operated such that the switch box 171 can be operated.

In this case, the control part 150 determines that the second heating block 122b is positioned in the standby position and controls the driving of the motor 131 such that it is possible to prevent the second heating block 122b from moving excessively.

In addition, as illustrated in FIG. 10, when the first heating block 122a and the second heating block 122b are changed from the standby position to the heating position through the driving of the driving part 130, the second heat insulating block 124b is moved together with the second heating block 122b such that the contact state between the second heat insulating block 124b and the roller 173 can be released.

The drawings and description exemplify a hinge roller-type limit switch as a position detection means for detecting the position of the second heating block 122b, but the present invention is not limited thereto, and various known limit switches, such as a lever-type limit switch, a pin-type limit switch and the like, may all be employed.

As another example, in the cell thawer 200 according to one embodiment of the present invention, the position detecting means may be a position detection sensor 270 for detecting the position of the heating blocks 122a, 122b that linearly move in the left and right directions through the driving of the driving part 130.

Such a position detection sensor 270 may be a non-contact position detection sensor.

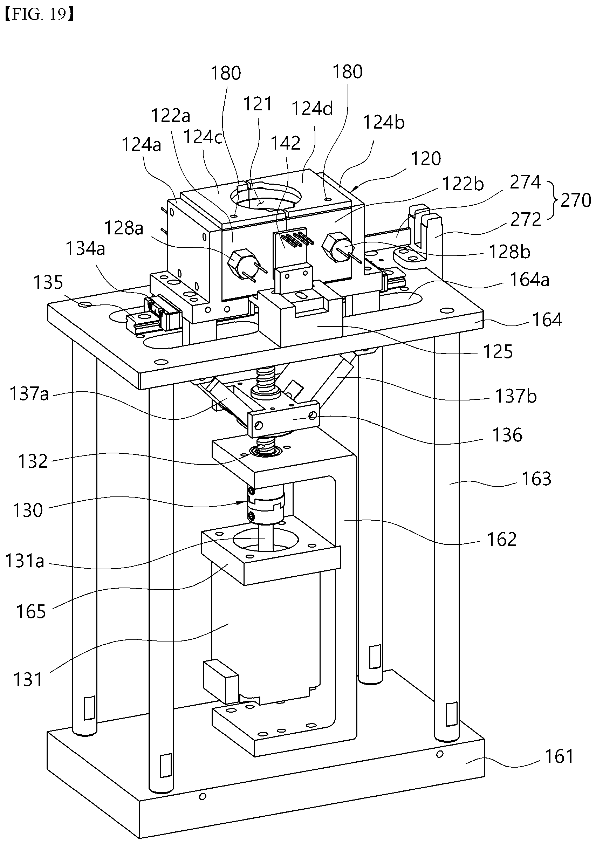

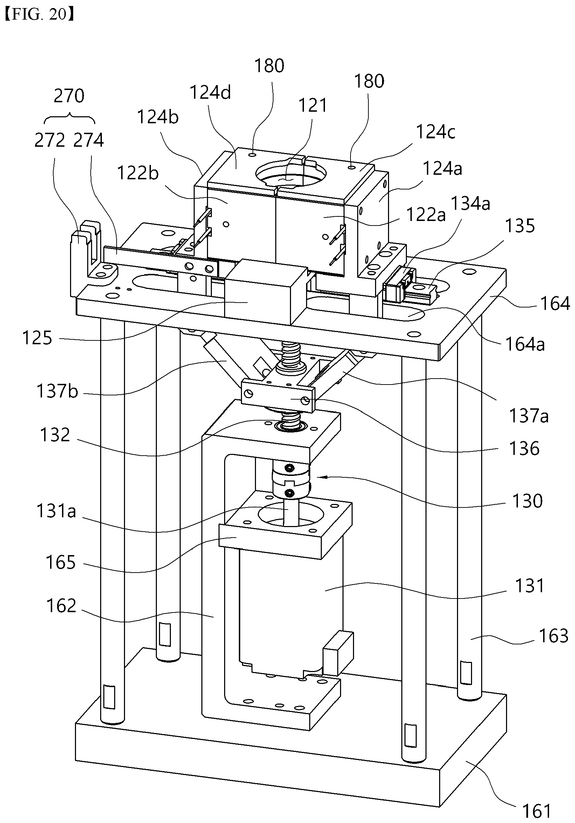

As a specific example, the non-contact position detection sensor 270 may be a known photo-microsensor. As a non-limiting example, as illustrated in FIGS. 19 and 20, the non-contact position detection sensor 270 may include a photosensor 272 which is installed on one surface of the support 164, and a detection bar 274, one end of which is fixedly installed in the second heat insulating block 124b.

Accordingly, when the detection bar 274 approaches the photosensor 272 through linear movement of the second heating block 122b and the second heat insulating block 124b, the photosensor 272 may detect the position of the second heating block 122b through the detection bar 274, and the information detected through the position detection sensor 270 may be provided toward the control part 150.

As a result, the control part 150 may accurately detect the position of the second heating block 122b through the non-contact position detection sensor 270, and may accurately check the origin of the motor 131.

Specifically, as described above, when the first heating block 122a and the second heating block 122b are changed from the heating position to the standby position through the driving of the driving part 130, the second heat insulating block 124b may move together with the second heating block 122b.

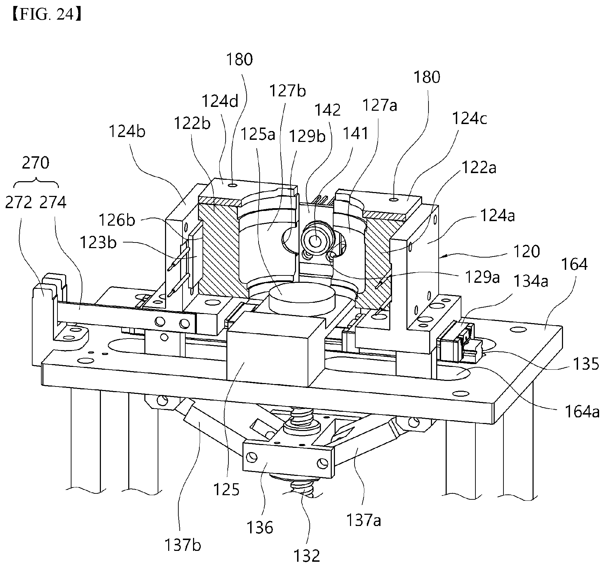

Through this, the detection bar 274 which is fixed to the second heat insulating block 124b may move toward the photosensor 272, and as illustrated in FIG. 24, when the second heating block 122b completely moves to the standby position, a partial length of the detection bar 274 may enter a groove part, which is formed in the photosensor 272.

In this case, the control part 150 determines that the second heating block 122b is positioned in the standby position and controls the driving of the motor 131 such that it is possible to prevent the second heating block 122b from moving excessively.

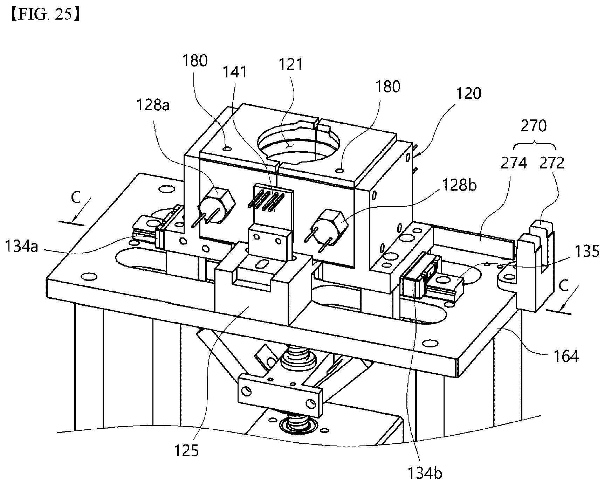

In addition, as illustrated in FIG. 25, when the first heating block 122a and the second heating block 122b are changed from the standby position to the heating position through the driving of the driving part 130, the detection bar 274 which is fixed to the second heat insulating block 124b may move away from the photosensor 272.

The drawings and description exemplify a non-contact sensor as a position detecting means for detecting the position of the second heating block 122b, but the present invention is not limited thereto, and various known position detection sensors may be employed as long as the position of the heating block 122 can be detected.

Meanwhile, the thawing part 120 may further include a sensor 141 for checking the state of the container 10 disposed in the heating space 121. For example, the sensor 141 may detect the surface temperature of the container 10 disposed in the heating space 121 and provide the detected result toward the control part 150.

Herein, the sensor 141 may be an infrared temperature sensor so as to measure the surface temperature of the container 10 in a non-contact manner, and the sensor 141 may measure the surface temperature of the container 10 which is disposed in the heating space while being fixed to a mounting member 142.

Accordingly, the control part 150 may output information about the container 10 that is detected through the sensor 141 to the display 118.

As a result, the user may check the thawing process of the biological material contained in the container 10 in real time through the information output from the display 118, and may monitor whether the biological material is thawing within an appropriate temperature range.

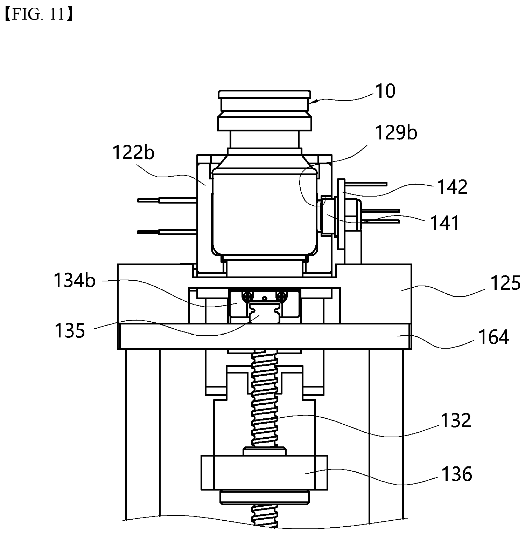

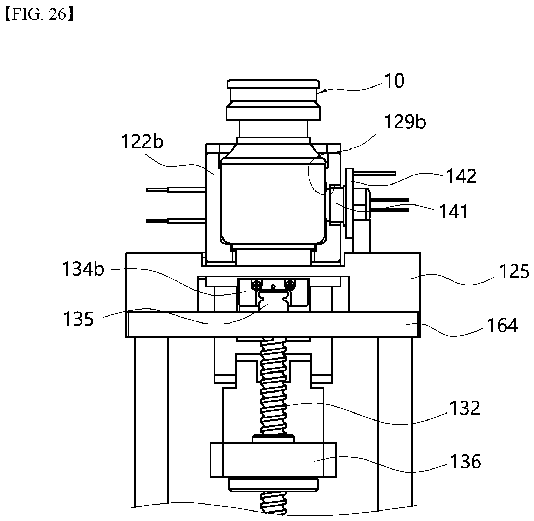

To this end, as illustrated in FIGS. 9, 11, 24 and 26, the sensor 141 may be coupled to the mounting member 142 to check the state of the container 10 disposed in the heating space 121, and the mounting member 142 may be coupled to the pedestal 125 which is fixedly coupled to the support 164.

In this case, the heating blocks 122a, 122b may include placement holes 129a, 129b which are formed in a shape corresponding to the sensor 141 such that the sensor 141 can enter the side of the heating space 121 side.

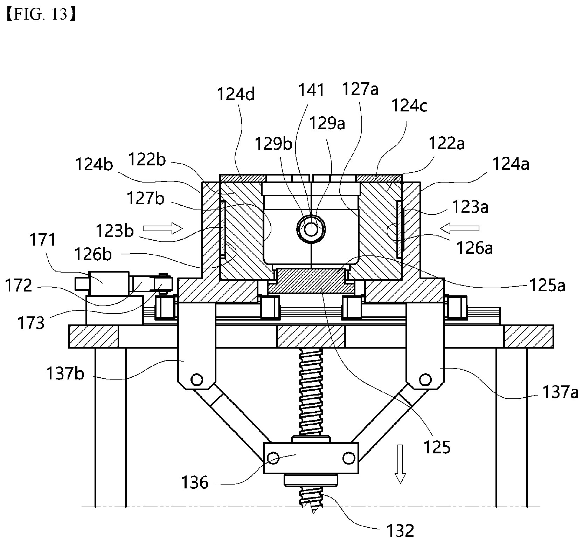

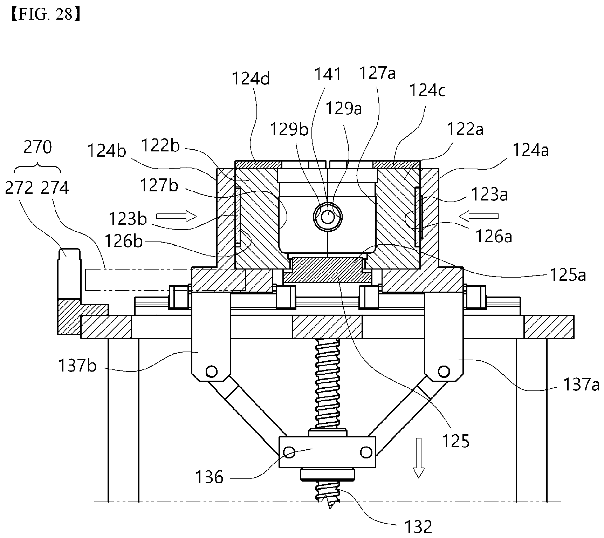

That is, the placement holes 129a, 129b may be formed to pass through the first heating block 122a and the second heating block 122b, respectively, as illustrated in FIGS. 13 and 28, and the placement holes 129a, 129b may be formed by joining a portion 129a which is formed on the first heating block 122a and a portion 129b which is formed on the second heating block 122b.

Accordingly, when the thawing part 120 is located at the heating position, the sensor 141 may measure the surface temperature of the container 10 at a very nearby location while not coming into contact with the container 10 that is disposed in the heating space 121 through the placement holes 129a, 129b.

Moreover, the temperature information measured by the sensor 141 may be used as information for detecting whether the container 10 is disposed in the heating space 121.

In the above description, an infrared temperature sensor is exemplified as the sensor 141, but the present invention is not limited thereto, and various known sensors may all be employed, and depending on the type of employed sensors, the information of the container 10 measured may vary.

Moreover, the mounting member 142 to which the sensor 141 is fixed may be detachably coupled to the pedestal 125. Accordingly, when the sensor 141 needs to be replaced, the sensor 141 may be easily replaced by separating the mounting member 142 from the pedestal 125.

The control part 150 may control the overall driving of the cell thawers 100, 200 according to one embodiment of the present invention.

As illustrated in FIG. 3, the control part 150 may include a circuit board 151 and a chipset 152 such as an MCU that are mounted on the circuit board 151, and the control part 150 may control all operations of the thawing part 120, the driving part 130, the sensor 141, the display 118, the operation part 117 and the position detection means described above.

In this case, when the container 10 accommodated in the heating space 121 is heated at the heating position, the control part 150 may control the driving of the driving part 130 so as to heat the container while the first heating block 122a and the second heating block 122b are in contact with each other, but it may also control the driving of the driving part 130 so as to heat the container while the first heating block 122a and the second heating block 122b are not in contact with each other.

That is, the control part 150 may set the heating position such that a predetermined gap is formed in the remaining portion except for portions where the opposing surfaces 127a, 127b for forming the heating space 121 are formed among surfaces on which the first heating block 122a and the second heating block 122b face each other.

Through this, the cell thawers 100, 200 according to one embodiment of the present invention may prevent a load that may be generated on the motor 131 in order to maintain contact between the first heating block 122a and the second heating block 122b at the heating position.

Additionally, in the process of thawing the biological material contained in the container 10 at the heating position, the cell thawers 100, 200 according to one embodiment of the present invention may discharge water vapor or moisture generated from the surface of the container 10 to the outside through the gap. Through this, the biological material may be thawed more smoothly.

Moreover, after the control part 150 preheats the heating blocks 122a, 122b to a predetermined temperature by driving the heaters 123a, 123b, it may start thawing the biological material contained in the container 10.

As described above, the control part 150 may maintain the temperature of the heating blocks 122a, 122b to be constant through PID control, and when the heating blocks 122a, 122b are provided in plurality, the control part 150 may individually control each of the heating blocks 122a, 122b.

Herein, the preheating temperature of the heating blocks 122a, 122b may be the same as the thawing temperature for thawing the biological material contained in the container 10 or may be a relatively higher temperature than the thawing temperature.

In addition, while the thawing of the container 10 introduced into the heating space 121 is completed and the heating blocks 122a, 122b are changed to the standby position, the control part 150 may maintain the temperature of the heating blocks 122a, 122b to be constant.

Through this, the cell thawers 100, 200 according to one embodiment of the present invention may be changed between a heating position and a standby position while the heating blocks 122a, 122b are continuously maintained at a constant temperature.

Accordingly, when the cell thawers 100, 200 according to one embodiment of the present invention are used, the user may sequentially insert and remove a plurality of containers 10 into the heating space 121 to perform the thawing, thereby minimizing the waiting time to increase work productivity.

Meanwhile, the cell thawer 200 according to one embodiment of the present invention may conveniently perform qualification evaluation to verify the operating temperature of the heating block.

To this end, the cell thawer 200 according to one embodiment of the present invention may further include a sensor insertion hole 180, and the sensor insertion hole 180 may be formed to be drawn inward from one surface of the heating blocks 122a, 122b at a certain depth.

The sensor insertion hole 180 may be a space into which a calibration sensor is inserted during calibration to check whether the heating blocks 122a, 122b are normally operating.

For example, the calibration sensor (not illustrated) may be a bar-shaped temperature sensor having a predetermined length, and a partial length of the calibration sensor may be inserted into the sensor insertion hole 180.

Accordingly, the verifier may conveniently measure the temperature of the heating blocks 122a, 122b through the calibration sensor inserted into the sensor insertion hole 180, and thus, it is possible to check whether the cell thawer 200 according to one embodiment of the present invention is operating normally.

Although the sensor insertion hole 180 may be provided as one, when the heating blocks 122a, 122b include the above-described first heating block 122a and second heating block 122b, it may be provided in plurality so as to be respectively formed in the first heating block 122a and the second heating block 122b.

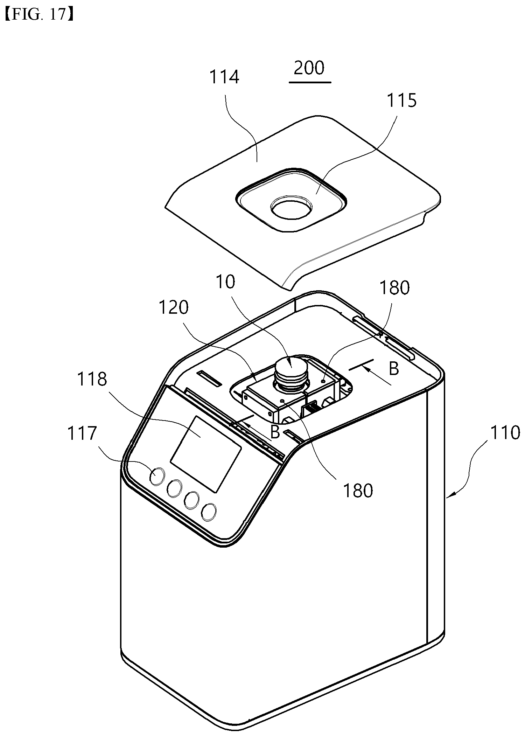

In this case, the sensor insertion hole 180 may be formed on a side surface of the heating blocks 122a, 122b, but as illustrated in FIG. 17, the sensor insertion hole 180 may be formed on the heating blocks 122a, 122b so as to be inserted from the upper surface to the lower portion of the heating block 122a, 122b at a certain depth.

In addition, when the cell thawer 200 according to one embodiment of the present invention includes auxiliary heat insulating blocks 124c, 124d for covering the upper surfaces of the heating blocks 122a, 122b, the sensor insertion hole 180 may be formed on the heating blocks 122a, 122b so as to be inserted from the upper surface to the lower portion of the heating blocks 122a, 122b to a certain depth while penetrating the auxiliary heat insulating blocks 124c, 124d.

Accordingly, when the upper cover 114 on which the inlet 115 is formed is separated from the housing 110, the sensor insertion holes 180 formed on the upper surfaces of the heating blocks 122a, 122b and the auxiliary heat insulating blocks 124c, 124d may be exposed to the outside.

As a result, even if only the upper cover 114 is separated from the housing 110 without the need to separate the entire housing in the cell thawer 200 according to one embodiment of the present invention, the sensor insertion hole 180 for calibration may be exposed to the outside.

Additionally, in the cell thawer 200 according to one embodiment of the present invention, since the sensor insertion hole 180 is formed to be inserted from the upper surface to the lower portion of the heating block 122a, 122b by a certain depth, the calibration sensor inserted into the sensor insertion hole 180 may maintain a state of being inserted upright into the sensor insertion hole 180 even without a separate fixing means.

Through this, after separating the upper cover 114, when the verifier inserts a partial length of the calibration sensor into the sensor insertion hole 180 formed on the upper surface of the heating blocks 122a, 122b, the verifier may simply and accurately measure the operating temperature of the heating blocks 122a, 122b.

In this case, the sensor insertion hole 180 may be formed to be positioned directly above the temperature sensors 128a, 128b embedded in the heating blocks 122a, 122b.

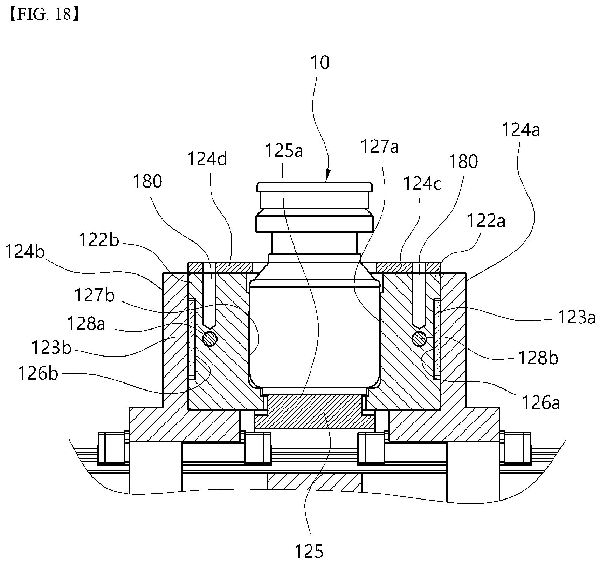

For example, as illustrated in FIG. 18, the sensor insertion hole 180 may be formed on the heating blocks 122a, 122b such that a lower end, which is a sealed end, is located directly above the temperature sensors 128a, 128b, and the lower end of the sensor insertion hole 180 may be formed on the heating block 122a or 122b to have a gap of 1 mm to 5 mm from the temperature sensor 128a or 128b.

Accordingly, during calibration, the temperatures of the heating blocks 122a, 122b measured by the calibration sensor inserted into the sensor insertion hole 180 may be similar to the temperatures of the heating blocks 122a, 122b measured by the temperature sensors 128a, 128b, when the cell thawer 200 according to one embodiment of the present invention operates.

As a result, when the calibration of the cell thawer 200 according to one embodiment of the present invention is performed by using the sensor insertion hole 180, the temperatures of the heating blocks 122a, 122b measured through the calibration sensor and the temperatures of the heating blocks 122a, 122b measured through the temperature sensors 128a, 128b during normal operation are similar, and thus, the calibration result may secure high reliability.

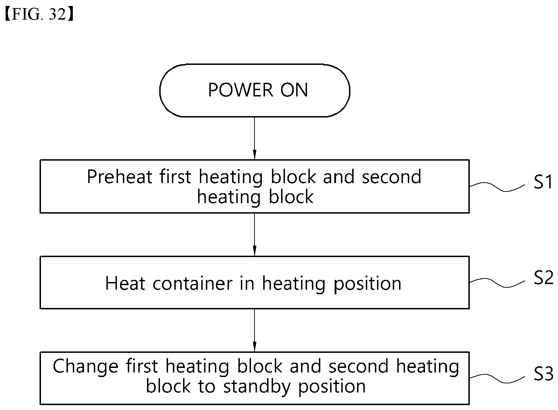

Hereinafter, the operation method of the above-described cell thawers 100, 200 will be described with reference to FIGS. 12 to 16 and FIGS. 27 to 32.

First of all, as a preparation step, the cover 116 for covering the inlet 115 is removed, and power is supplied to the cell thawers 100, 200.

When power is applied to the cell thawers 100, 200, the control part 150 may drive the driving part 130 to change the thawing part 120 to a standby position.

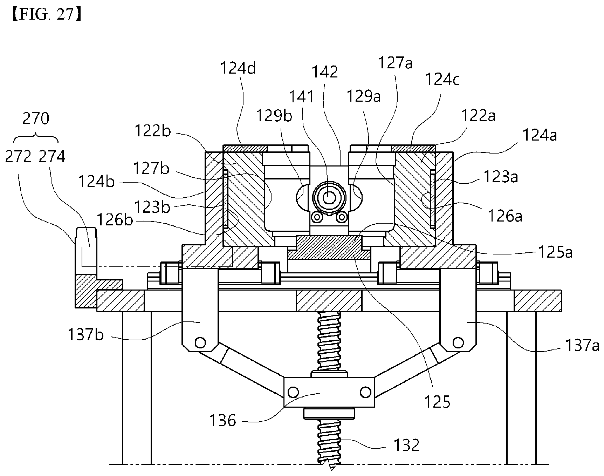

That is, as illustrated in FIGS. 12 and 27, the control part 150 may drive the motor 131 to raise the moving member 136. Accordingly, the first heating block 122a and the second heating block 122b may be slidably moved in a direction of moving away from each other, and may be changed to a standby position of being disposed to be spaced apart from each other at a certain interval.

If the first heating block 122a and the second heating block 122b are located in the standby position, the preparation step of changing the thawing part 120 to the standby position when power is applied may be omitted.

Next, as a first step (S1), the control part 150 operates the first heater 123a and the second heater 123b to preheat the first heating block 122a and the second heating block 122b to a predetermined temperature.

For example, the preheating temperature of the first heating block 122a and the second heating block 122b may be equal to or higher than the thawing temperature for thawing the biological material contained in the container 10.

In this case, the preheating of the first heating block 122a and the second heating block 122b may be performed in a state where surfaces on which the first heating block 122a and the second heating block 122b face each other are in contact with each other.

That is, as illustrated in FIGS. 13 and 28, the control part 150 may lower the moving member 136 by driving the motor 131. Accordingly, the first heating block 122a and the second heating block 122b may be slidably moved in a direction of moving closer to each other in the standby position, respectively, and the first heating block 122a and the second heating block 122b may be changed to a state in which surfaces on which the first heating block 122a and the second heating block 122b face each other are in contact with each other.

In this state, the control part 150 may control the driving of the motor 131 such that the contact state of the first heating block 122a and the second heating block 122b can be maintained until the preheating of the first heating block 122a and the second heating block 122b is finished.

Accordingly, while the first heating block 122a and the second heating block 122b are in contact with each other, the first heating block 122a and the second heating block 122b may be heated simultaneously through the first heater 123a and the second heater 123b disposed on both sides, respectively, and thus, the first heating block 122a and the second heating block 122b may be quickly heated to a predetermined temperature through heat transferred from the first heater 123a and the second heater 123b.

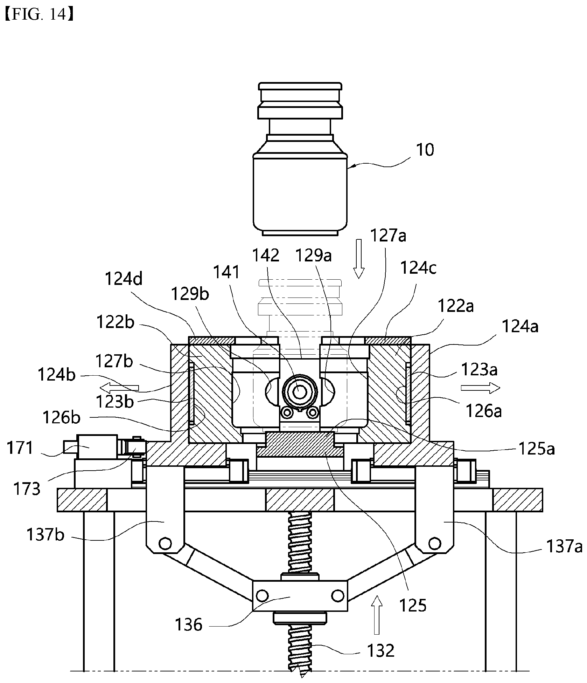

Thereafter, when the preheating of the first heating block 122a and the second heating block 122b is completed, the control part 150 may output a message indicating that the preheating is completed to the display 118, and the control part 150 may slide and move the first heating block 122a and the second heating block 122b by driving the motor 131. Through this, as illustrated in FIG. 14, the first heating block 122a and the second heating block 122b may be changed to a standby position of being disposed to be spaced apart from each other at a predetermined interval.

In this way, when the preheating of the first heating block 122a and the second heating block 122b is completed, the user checks the set thawing time through the display 118 and, if the set thawing time needs to be changed, the user may change the set thawing time to an appropriate time by manipulating the manipulation part 117.

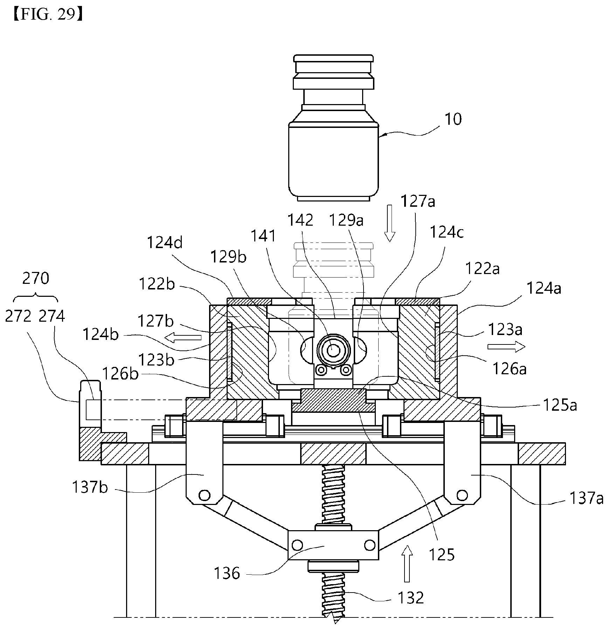

Next, as illustrated in FIGS. 14 and 29, while the first heating block 122a and the second heating block 122b are located in the standby position, the user may place the container 10 requiring thawing between the first heating block 122a and the second heating block 122 disposed in the standby position. In this case, the container 10 may be disposed such that the lower surface thereof is seated on one surface of the protruding member 125a.

Thereafter, the user may send a thawing start signal through the manipulation part 117 while the container 10 requiring thawing is placed between the first heating block 122a and the second heating block 122b that are disposed in the standby position.

Accordingly, the control part 150 may heat the container 10 to thaw the biological material contained in the container 10 as a second step (S2).

That is, the control part 150 may lower the moving member 136 by driving the motor 131 such that the first heating block 122a and the second heating block 122b can be moved in a direction of moving closer to each other.

Accordingly, as illustrated in FIGS. 15 and 30, the first heating block 122a and the second heating block 122b may be changed to a heating position for forming a heating space 121 for accommodating and heating the container 10 in the standby position, and the circumferential surface of the container 10 may be surrounded by the first heating block 122a and the second heating block 122b while the container 10 is disposed in the heating space 121.

In addition, the sensor 141 may measure the surface temperature of the container 10 at a very close position without contacting the container 10 that is disposed in the heating space 121 through the placement holes 129a, 129b.

Through this, the container 10 may be heated by the thawing time set by the user through the heat transferred from the first heating block 122a and the second heating block 122b, and through the sensor 141, it is possible to measure the state of the container 10 in real time.

In this case, the control part 150 may output a message indicating that it is thawing to the display 118, and based on the temperature information transmitted from the first temperature sensor 128a and the second temperature sensor 128b, the temperatures of the first heating block 122a and the second heating block 122b may be maintained to be constant by controlling the operation of the first heater 123a and the second heater 123b, and the state information about the container 10 detected through the sensor 141 may be output to the display 118 through the driving of the control part 150.

In this case, as described above, while the container 10 disposed in the heating space 121 is not in contact with the first heating block 122a and the second heating block 122b, it may be heated by the heat transferred from the first heating block 122a and the second heating block 122b in a non-contact manner.

That is, while the container 10 is inserted into the heating space 121, the circumferential surface of the container 10 may be spaced apart by a certain interval from the opposing surface 127a of the first heating block 122a and the opposing surface 127b of the second heating block 122b forming the heating space 121.

Through this, a gap (d) may be formed between the pair of opposing surfaces 127a, 127b and the outer surface of the container 10 while the container 10 is disposed in the heating space 121.

Accordingly, the heat stored in the first heating block 122a and the second heating block 122b may be transferred to the relatively low-temperature container 10 through convection and/or radiation instead of conduction.

As a result, since the circumferential surface of the container 10 may be heated through convective heat and/or radiant heat, the circumferential surface of the container 10 may be uniformly heated through convective heat and/or radiant heat transferred from the heating blocks 122a, 122b.

Additionally, in the second step (S2), the control part 150 may control the motor 131 to heat the container 10 in a state where the first heating block 122a and the second heating block 122b are in contact with each other, but it is possible to control the driving of the motor 131 to heat the container 10 in a state where the first heating block 122a and the second heating block 122b do not contact each other.

That is, the control part 150 may set the heating position to form a predetermined gap in the remaining portion except for portions where the opposing surfaces 127a, 127b for forming the heating space 121 are formed among the surfaces on which the first heating block 122a and the second heating block 122b face each other.

Through this, the cell thawers 100, 200 according to one embodiment of the present invention may prevent a load that may occur in the motor in order to maintain a state in which the first heating block 122a and the second heating block 122b are in contact with each other in the heating position, and in the process of thawing the biological material contained in the container 10 at the heating position, water vapor or moisture generated from the surface of the container 10 may be discharged to the outside through the gap such that it is possible to thaw the biological material more smoothly.

Next, when the thawing time set by the user elapses, the control part 150 may output a message indicating completion to the display 118, and as a third step (S3), the control part 150 may drive the motor 131 to change the first heating block 122a and the second heating block 122b to a standby position.

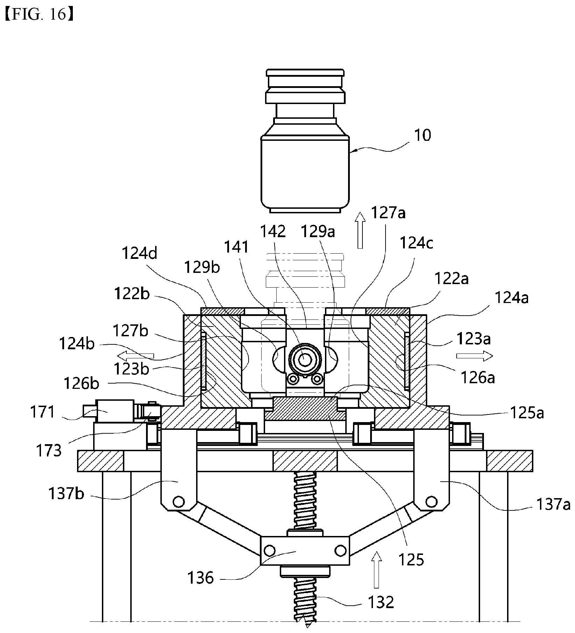

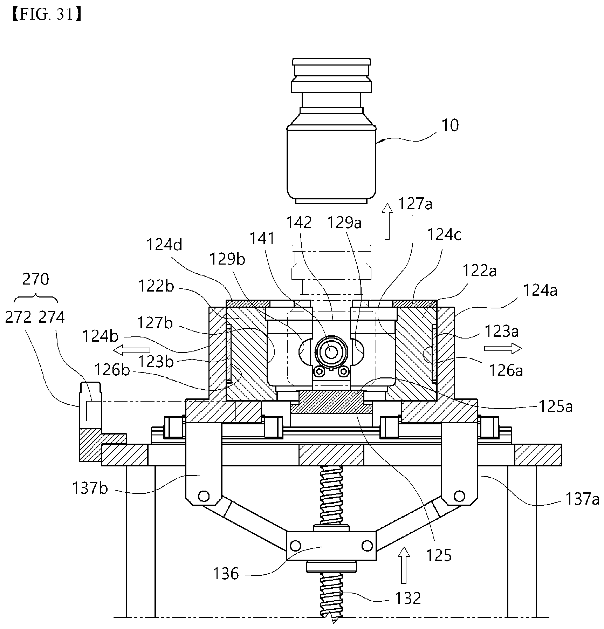

Accordingly, as illustrated in FIGS. 16 and 31, the user may remove the container 10 that is disposed on one surface of the protruding member 125a in a state where the first heating block 122a and the second heating block 122b are located in the standby position.

In this case, the control part 150 may control the operation of the first heater 123a and the second heater 123b such that the temperatures of the first heating block 122a and the second heating block 122b are continuously maintained to be constant.

Accordingly, the user may sequentially thaw a plurality of containers 10 by sequentially repeating the first step (S 1) and the second step (S2).

Although one exemplary embodiment of the present invention has been described above, the spirit of the present invention is not limited to the exemplary embodiments presented herein, and those skilled in the art who understand the spirit of the present invention may easily suggest other exemplary embodiments by changing, modifying, deleting or adding components within the scope of the same spirit, but this will also fall within the scope of the present invention.

## Claims

1. A cell thawer, comprising:
a thawing part comprising a heating block which comprises a first heating block and a second heating block that form a heating space for heating a container storing a predetermined amount of a biological material including cells, a heater which is installed in the heating block to provide heat to the heating block, a heat insulating block which is coupled to the heating block so as to encompass the heater, and a pedestal which is for supporting a lower portion of the container in the heating space;
a driving part for providing a driving force that linearly moves each of the first heating block and the second heating block in the left and right directions; and
a control part for controlling the operations of the thawing part and the driving part.

2. The cell thawer of claim 1, wherein the container accommodated in the heating space is heated by heat transferred from the heating block in a non-contact manner without contacting the heating block.

3. The cell thawer of claim 1, wherein the heating block is made of a thermally conductive material.

4. The cell thawer of claim 1, wherein the heating block comprises an arrangement groove which is formed on one surface to be drawn inward, the heater is inserted into the arrangement groove, and the heat insulating block is coupled to the heating block to cover the arrangement groove.

5. The cell thawer of claim 1, wherein the heating space is formed through opposing surfaces that are each formed to be drawn inward to have a shape corresponding to the circumferential surface of the container on surfaces on which the first heating block and the second heating block face each other.

6. The cell thawer of claim 1, wherein the pedestal comprises a protruding member that is formed to protrude from one surface at a certain height, and the protruding member supports a lower surface of the container.

7. The cell thawer of claim 1, wherein the thawing part further comprises an auxiliary heat insulating block for covering an upper surface of the heating block.

8. The cell thawer of claim 1, wherein the thawing part comprises a temperature sensor which is installed on the heating block to measure the temperature of the heating block.

9. The cell thawer of claim 8, wherein the cell thawer further comprises a sensor insertion hole which is formed to be drawn at a certain depth from an upper surface of the heating block to a lower portion such that a calibration sensor for measuring the temperature of the heating block can be inserted therein, and
wherein the sensor insertion hole is formed to be located directly above the temperature sensor.

10. The cell thawer of claim 1, wherein the driving part comprises a motor for providing a driving force, a rotating member which is axially coupled to a rotating shaft of the motor, a moving member which moves up and down along the rotating member when the rotating member rotates, and a power transmission member for interconnecting the moving member and the thawing part such that the first heating block and the second heating block can be moved linearly in the left and right directions, respectively, through a driving force of the motor.

11. The cell thawer of claim 10, wherein the power transmission member comprises a guide block which is fixedly coupled to the heat insulating block, a guide rail which guides the linear movement of the guide block, and a link member which links the guide block and the moving member to each other.

12. The cell thawer of claim 1, wherein the cell thawer further comprises a sensor for detecting the state of the container placed in the heating space.

13. The cell thawer of claim 1, wherein the cell thawer further comprises a limit switch for detecting the position of the heating block through contact with the heat insulating block.

14. The cell thawer of claim 1, wherein the cell thawer further comprises a non-contact position detection sensor for detecting the position of the heating block.

15. The cell thawer of claim 1, wherein the cell thawer comprise a housing having an inlet which is formed through a position corresponding to the heating space.

16. The cell thawer of claim 15, wherein the cell thawer further comprises a sensor insertion hole which is formed to be drawn at a certain depth from an upper surface of the heating block to a lower portion such that a calibration sensor for measuring the temperature of the heating block can be inserted therein,
wherein the housing comprises a box-shaped main body and an upper cover that is detachably coupled to the main body so as to cover an upper portion of the main body,
wherein the inlet is formed on the upper cover so as to be located in a position corresponding to the heating space, and
wherein the sensor insertion hole is exposed to the outside when the upper cover and the main body are separated.

17. The cell thawer of claim 15, wherein the cell thawer comprises a communication port which is provided on one side of the housing.

18. The cell thawer of claim 1, wherein when heating a container accommodated in the heating space, the control part controls the driving of the driving part to heat the container in a state where the first heating block and the second heating block are not in contact with each other.

19. The cell thawer of claim 1, wherein the control part individually controls the temperatures of the first heating block and the second heating block.

20. A method for operating a cell thawer comprising a first heating block and a second heating block that form a heating space for accommodating a container storing a predetermined amount of a biological material including cells through linear movement by a driving force of a motor, and heating the container accommodated in the heating space through heat provided from a heater, the method comprising:
a first step of preheating the first heating block and the second heating block to a predetermined temperature by driving the heater while one surface of the first heating block and one surface of the second heating block facing each other are in contact with each other;
a second step of heating the container which is placed in the heating space so as to thaw the biological material while the first heating block and the second heating block are changed to a heating position forming the heating space; and
a third step of changing the first heating block and the second heating block in a direction of moving away from each other from the heating position to a standby position such that the container placed in the heating space can be removed,
wherein in the second step, the container placed in the heating space is heated by heat transferred from the first heating block and the second heating block in a non-contact manner while not contacting the first heating block and the second heating block.
